**Europäisches Patentamt**

**European Patent Office**

**Office européen des brevets**

(11) Veröffentlichungsnummer: **0 032 751**
**B1**

(12) # EUROPÄISCHE PATENTSCHRIFT

(45) Veröffentlichungstag der Patentschrift:
**18.04.84**

(51) Int. Cl.³: **A 61 B 10/00**

(21) Anmeldenummer: **81100426.6**

(22) Anmeldetag: **21.01.81**

(54) Mechanisches Zusatzgerät zu Compoundgeräten für Echomammographie.

(30) Priorität: **21.01.80 DE 3002067**
**07.11.80 DE 3042079**

(43) Veröffentlichungstag der Anmeldung:
**29.07.81 Patentblatt 81/30**

(45) Bekanntmachung des Hinweises auf die Patenterteilung:
**18.04.84 Patentblatt 84/16**

(84) Benannte Vertragsstaaten:
**AT BE CH FR GB IT LI NL SE**

(56) Entgegenhaltungen:
**WO - A - 80/00193**
**DE - A - 2 919 995**
**GB - A - 2 015 732**
**US - A - 3 480 002**
**US - A - 4 105 018**

**Die Akte enthält technische Angaben, die nach dem Eingang der Anmeldung eingereicht wurden und die nicht in dieser Patentschrift enthalten sind.**

(73) Patentinhaber: **Igl, Wolfgang Dr., Linprunstrasse 35, D-8000 München 2 (DE)**

(72) Erfinder: **Igl, Wolfgang Dr., Linprunstrasse 35, D-8000 München 2 (DE)**

(74) Vertreter: **Wilheims, Rolf E., Dr. et al, WILHELMS & KILIAN Patentanwälte Eduard-Schmid-Strasse 2, D-8000 München 90 (DE)**

Mechanisches Zusatzgerät zu Compoundgeräten für Echomammographie

Die Erfindung betrifft ein mechanisches Zusatzgerät zu Compoundgeräten für Echomammographie zur Ultraschalluntersuchung der weiblichen Brust, bestehend aus einem Wasserbehälter mit einer Abdeckung, die eine Öffnung aufweist; ferner einem in den Wasserbehälter unter die Öffnung ragenden Haltearm, der mindestens einen Schallkopf, dessen Schallfeld-Ausbreitungsvektor auf die Öffnung gerichtet ist, aufweist, und der Rotationsbewegungen um wenigstens eine Achse ausführen kann.

Eine derartige Vorrichtung ist aus der GB-A-2 015 732 bekannt. Bei dieser Vorrichtung ist der im wesentlichen horizontal ausgerichtete Haltearm auf einer senkrecht zur Ebene der Öffnung ausgerichteten Achse 4 befestigt, und um diese Achse drehbar ausgebildet. Ausserdem ist der Haltearm um eine weitere, zur ersten Achse senkrecht und unterhalb der Ebene der Öffnung angeordnete Achse kippbar. Schliesslich ist der Schallkopf entlang des Haltearms linear beweglich angeordnet.

Nachteilig an dieser Vorrichtung ist der Umstand, dass mit ihr keine Schnittbildserien erzeugt werden können, bei denen der Schallkopf immer senkrecht auf das Zielorgan gerichtet ist. Darüber hinaus ist es nachteilig, dass nur unter grossem apparativen und steuertechnischen Aufwand Schnittbildserien erzeugt werden können, deren Drehachse durch die Brustwarze verläuft; dieses ist jedoch wünschenswert, weil sich dann vorteilhafterweise halbrunde Schnittbilder ergeben, die die Untersuchung erheblich erleichtern, wobei dieses ausserdem den anatomischen Gegebenheiten entspricht, weil alle Milchgänge sternförmig auf die Brustwarzen zu verlaufen. Schliesslich ist die bekannte Vorrichtung konstruktiv insofern aufwendig, als bei optimaler Benutzung der Vorrichtung eine Drehbewegung, eine Kippbewegung und eine Linearbewegung steuertechnisch harmonisiert werden müssen.

Aus der US-A-4 105 018 ist eine ähnliche Vorrichtung bekannt, bei der der Schallkopf jedoch lediglich um eine vertikale Drehachse 32 durch den Mittelpunkt der Öffnung drehbar angeordnet ist; damit treten alle bereits eingangs geschilderten Nachteile bei dieser Vorrichtung ebenfalls auf; darüber hinaus handelt es sich hierbei im Gegensatz zum vorliegenden Anmeldungsgegenstand um eine Vorrichtung oder ein Verfahren zur Durchschallung.

Die aus der DE-OS 2 919 995 bekannte Ultraschall-Diagnosevorrichtung weist einen Schallkopf auf, der konstruktionsbedingt ebenfalls nur um eine senkrechte Achse drehbar ist, welche sich durch den Boden des Behälters hindurch erstreckt. Auch an dieser Vorrichtung ist der Umstand nachteilig, dass mit ihr keine Schnittbildserien erzeugt werden können, bei denen der Schallkopf immer senkrecht auf das Zielorgan gerichtet ist, und dass keine Schnittbildserien erzeugt werden können, deren Drehachse durch die Brustwarze verläuft.

Der vorliegenden Erfindung liegt die Aufgabe zu Grunde, unter Vermeidung der aufgezeigten Nachteile der bekannten Vorrichtungen aus dem Stand der Technik, eine solche der eingangs geschilderten Art zur Verfügung zu stellen, mit deren Hilfe schnelle, genaue und reproduzierbare Untersuchungsergebnisse erhalten werden können. Insbesondere soll die Vorrichtung gegenüber den bekannten Vorrichtungen hinsichtlich der Bauweise vereinfacht sein. Insbesondere sollen auch Sonogrammschnittbildserien von einer weiblichen Brust erstellbar sein können, deren Drehachse durch die Brustwarze verläuft, so dass sich eine Serie von halbrunden Schnittbildern ergibt. Schliesslich soll die erfindungsgemässe Vorrichtung derart ausgebildet sein, dass der Abstand zwischen Zielobjekt und Schallkopf praktisch immer der gleiche sein kann.

Die erfindungsgemässen Aufgaben werden durch das kennzeichnende Merkmal der Vorrichtung gemäss Anspruch 1 gelöst.

Weitere vorteilhafte Ausgestaltungen der Erfindung ergeben sich aus den Ansprüche 2 bis 5.

Die erfindungsgemässe Vorrichtung gemäss Anspruch 1 erlaubt somit eine praktisch vollautomatische Untersuchung der weiblichen Brust, wobei sich die Patientin auf die gegebenenfalls in einer Liege integrierte Vorrichtung gemäss Anspruch 1 derart legt, dass die zu untersuchende Brust durch die mit abgerundeten Kanten versehene Öffnung der Abdeckung in das Wasserbad hängt und deren Hautoberfläche allseitig von Wasser ohne Verwendung einer Folie umgeben ist. Der im Wasser unter der Öffnung angeordnete Haltearm mit mindestens einem Schallkopf führt, mechanisch oder elektrisch angetrieben, eine halbkreisartige Rotationsbewegung aus, deren Achse etwa in der Ebene der Öffnung liegt und durch den Mittelpunkt der Öffnung geht oder die einige Zentimeter senkrecht oberhalb des Mittelpunkts der Ebene der Öffnung im Thoraxinnenraum der Patientin liegt, um Überschneidungen der Schallimpulse in der Abbildung der zu untersuchenden Brust zu vermeiden. Bei einer derartigen Rotationsbewegung des den Schallkopf tragenden Haltearms um maximal um etwa 140° - 180° wird ein Schichtbild in einer Ebene erzeugt, wobei das in an sich bekannter Weise erzeugte Sonogrammschnittbild gegebenenfalls auf Magnetband oder -platte gespeichert wird. Um mehrere Sonogrammschnittbilder zu erhalten, die zu einer Gesamtuntersuchung der Brust notwendig sind, kann nach jeder halbkreisförmigen Rotationsbewegung des den Schallkopf tragenden Haltearms die Stellung des Schallkopfes in Bezug auf das Zielorgan (weibliche Brust) durch geringfügige Verschiebung des Schallkopfes senkrecht zur Rotationsebene des Haltearms verschoben werden.

Bevorzugt wird hierbei der den Schallkopf tragende Bereich des Haltearms senkrecht zur Rotationsebene des unter die Öffnung ragenden Haltearms bewegbar ausgebildet, und zwar vorzugsweise teleskopartig und hydraulisch. Auf diese Weise kann die Patientin während der Gesamtuntersuchung entspannt auf der Vorrichtung liegen.

Die Koordination der Rotationsbewegung des Haltearms und das hydraulische teleskopartige Ausfahren des den Schallkopf tragenden Bereichs des Haltearms erfolgt elektronisch derart, dass nach jeder vollzogenen Rotationsbewegung um etwa 140° - 180°C der den Schallkopf tragende Bereich des Haltearms um wenige Millimeter hydraulisch ausgefahren wird, bevor der Haltearm die nächste Rotationsbewegung um 140° - 180° durchführt. Im folgenden wird anstelle von Rotationsbewegung bzw. -ebene stets der Ausdruck Schwingbewegung bzw. -ebene benutzt.

Schliesslich kann der Haltearm senkrecht zur Ebene der Öffnung höhenverstellbar ausgebildet sein, wodurch gewährleistet wird, dass vor Beginn der Untersuchung die optimale Distanz zwischen Schallkopf und weiblicher Brust eingestellt werden kann.

Mit den geschilderten erfindungsgemässen Vorrichtungen werden Serien von Sonogrammschnittbildern erhalten, die zueinander parallel sind. Um die exakten Lagekoordinaten eines einzelnen Sonogrammschnittbildes festzuhalten, wird, gekoppelt mit der Aufzeichnung der Sonogrammschnittbilder, die Ausfahrposition des Haltearms gleichzeitig aufgezeichnet; Einrichtungen dieser Art sind den Fachleuten geläufig und nicht Gegenstand der vorliegenden Erfindung.

Bei einer bevorzugten Ausführungsform ist senkrecht unterhalb der Ebene der Öffnung eine Videokamera zur Aufzeichnung der jeweiligen Schallschnittebene im Verhältnis zu einem auf der Haut der Patientin eventuell mit Filzstift angezeichneten Tastbefund im Bereich der Brust während der Messungen angeordnet, wobei das Videobild gleichzeitig mit dem jeweiligen Sonogrammschnittbild, bevorzugt in Überlagerung mit dem jeweiligen Sonogrammschnittbild, aufgezeichnet wird.

Bei einer anderen bevorzugten Ausführungsform sind auf dem Haltearm zwei oder mehrere Schallköpfe, ein linear array-Multielement oder ein Multielement Ring-Transclucer angeordnet. Werden sie in paralleler Schallrichtung angeordnet, werden gleichzeitig mehrere parallel zueinander liegende Sonogrammschnittbilder erhalten und aufgezeichnet, wodurch sich die Untersuchungsdauer erheblich verkürzt. Hierbei sind die Schallköpfe auf dem Haltearm senkrecht zur Schwingebene des Haltearms gegeneinander versetzt angeordnet.

Bei einer anderen erfindungsgemässen Ausführungsform können mehrere Schallköpfe nebeneinander auf dem Haltearm angeordnet sein, wodurch ermöglicht wird, dass derselbe Schnitt mit verschiedenen Schallfrequenzen erhalten werden kann.

Schliesslich können bevorzugt die Schallköpfe gegeneinander in Schallrichtung gewinkelt auf dem Haltearm angeordnet sein, wodurch Darstellung und Untersuchung räumlicher Strukturen erheblich verbessert werden kann.

Während bei den zuvor beschriebenen Ausführungsformen gemäss der Erfindung zueinander parallele Sonogrammschnittbilder erhalten werden, werden mit einer anderen erfindungsgemässen bevorzugten Ausführungsform Serien von Sonogrammschnittbildern erhalten, die um jeweils den gleichen Winkelbetrag um eine gemeinsame Achse gegeneinander verdreht sind.

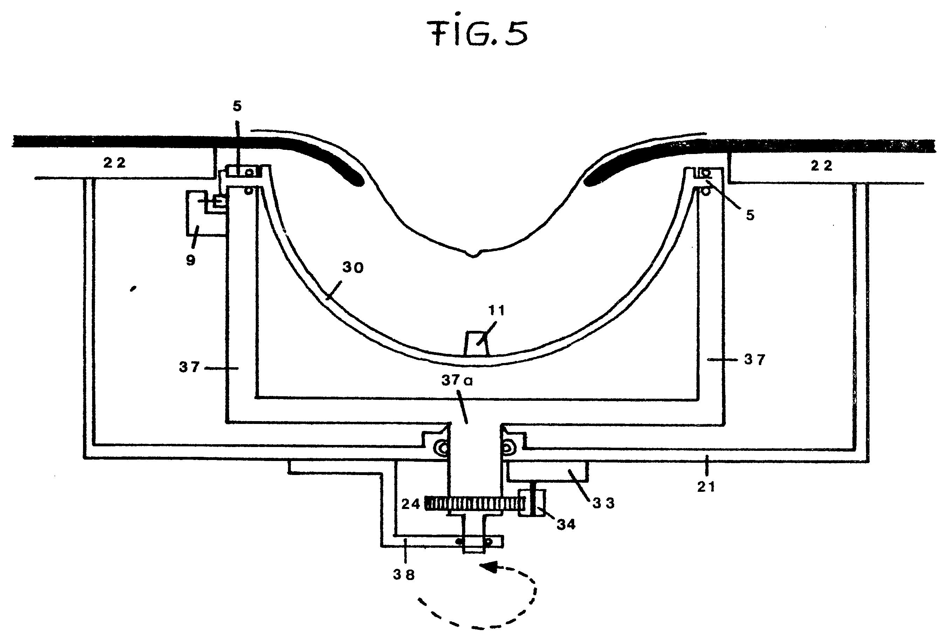

Hierzu wird bei einer besonderen Ausführungsform der erfindungsgemässen Vorrichtung das Lager, in dem der Haltearm zur Ausführung der etwa halbkreisförmigen Schwingbewegungen um den Mittelpunkt der Öffnung in der Abdeckung des Wasserbehälters herum gelagert ist, zumindest halbkreisförmig konzentrisch um die Öffnung herum verstellbar ausgebildet. Bevorzugt werden die Lager des Schallarms auf einer kreisförmigen Schiene, die konzentrisch um die Öffnung in der Abdeckung verläuft, drehbar ausgebildet. Insbesonders bevorzugt ist jedoch die Ausführungsform nach dem Anspruch 11 und der Figur 5.

Besonders vorteilhaft bei den letzteren Ausführungsformen ist der Umstand, dass die Drehachse der Sonogrammschnittbilder durch die Brustwarze verläuft, so dass sich vorteilhafterweise halbrunde Schnittbilder ergeben, die die Begutachtung erheblich erleichtern. Darüber hinaus entspricht dieses Prinzip den automatischen Gegebenheiten, weil alle Milchgänge sternförmig auf die Brustwarzen zu laufen. Damit ist es einfacher, einen erweiterten Milchgang im Längsschnitt abzubilden. Schliesslich wird durch diese Ausführungsform die Mechanik und damit auch das gesamte Gerät vereinfacht.

Die Erfindung wird anhand der folgenden Figuren näher erläutert.

Fig. 1 ist ein Querschnitt durch eine erfindungsgemässe Ausführungsform,

Fig. 2 ist der Querschnitt durch eine andere erfindungsgemässe Ausführungsform,

Fig. 3 ist eine Ausschnittsvergrösserung im Querschnitt durch den entsprechenden Vorrichtungsteil gemäss Fig. 2,

Fig. 4 ist eine Ausschnittsvergrösserung im Querschnitt durch den entsprechenden Vorrichtungsteil gemäss Fig. 2,

Fig. 5 ist eine weitere erfindungsgemässe Ausführungsform,

Fig. 6 ist eine Weiterbildung der erfindungsgemässen Ausführungsform gemäss Fig. 5,

Fig. 7 ist ein schematischer Querschnitt und eine Aufsicht auf eine erfindungsgemäss ausgebildete Abdeckung des mit Wasser gefüllten Wasserbehälters,

Fig. 8 ist eine weitere Ausführungsform der Vorrichtung gemäss Fig. 6.

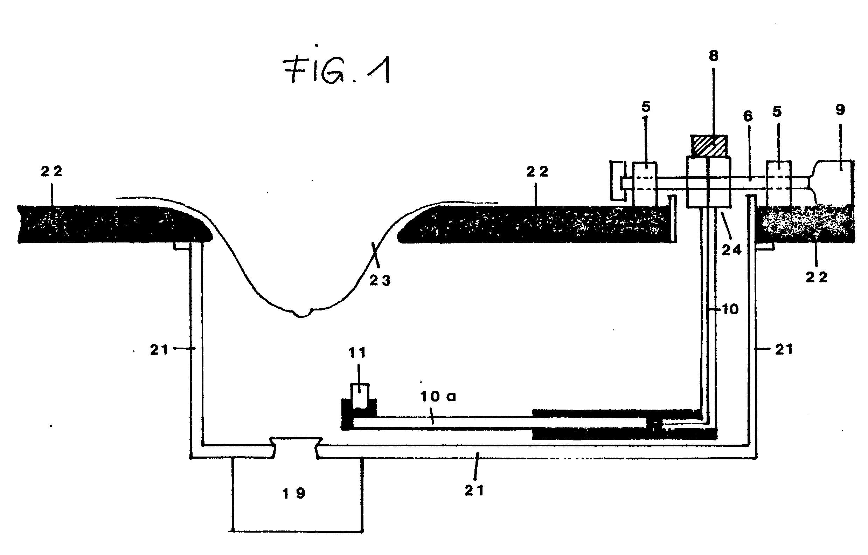

Die Vorrichtung gemäss Fig. 1 besteht aus einem Wasserbehälter 21, der rund oder eckig ausgebildet sein kann. Der Wasserbehälter ist mit einer Abdeckung 22 versehen, die eine Öffnung 23 aufweist, durch die der Busen der Patientin während der Untersuchung in den mit Wasser gefüllten Wasserbehälter 21 eintaucht. Durch eine weitere Öffnung 24 in der Abdeckung 22 ragt ein Haltearm 10 in den Wasserbehälter 21 hinein, wobei auf dem vorderen Bereich 10a, der sich im Wasserbehälter 21 unter der Öffnung 23 befindet, mindestens ein Schallkopf 11, der auf die Öffnung 23 gerichtet ist, angeordnet ist. Der vordere Bereich 10a des Haltearms 10 kann aus diesem hydraulisch teleskopartig aus- und eingefahren werden, wobei der Vorschub stufenlos oder in vorgegebenen Millimeterstufen erfolgen kann und mindestens dem Durchmesser der Öffnung 23 entspricht. Oberhalb der Öffnung 24 sind an deren Rand zwei Lager 5 angeordnet, in der die Welle 6, die Öffnung 24 überbrückend, drehbar gelagert ist. Im Bereich der Öffnung 24 ist die Welle 6 mit dem noch freien Ende des Haltearms 10 im wesentlichen starr verbunden, so dass bei einer Drehung der Welle 6 um einen bestimmten Winkel der Haltearm 10 mit dessen vorderem Teil 10a und dem darauf angeordneten Schallkopf 11 eine Schwingbewegung um den gleichen Winkelbetrag ausführt. Die Welle 6 wird durch einen Motor 9 derart angetrieben, dass der Haltearmteil 10a mit dem Schallkopf 11 eine nahezu halbkreisförmige Schwingbewegung um etwa 140° - 180° innerhalb des Wasserbehälters 21 ausführt. Während einer solchen nahezu halbkreisförmigen Schwingbewegung des Haltekopfes 11 um den durch die Öffnung 23 hängenden Busen der Patientin herum wird ein Sonogrammschnittbild aufgenommen und gespeichert. Durch einen vorgegebenen Vorschub, z.B. im Bereich von 1 bis 5 mm, wird eine andere Position des Schallkopfs 11 in Bezug auf das Zielorgan erreicht, und ein weiteres zu dem vorhergehenden paralleles Sonogrammschnittbild erhalten und aufgezeichnet. Auf diese Weise kann eine Serie von zueinander parallelen Sonogrammschnittbildern des Zielorgans erhalten und aufgezeichnet werden, die sofort oder später zur Strukturuntersuchung des Zielorgans dient.

Vorzugsweise ist der Haltearm 10 mit der Welle 6 im wesentlichen fest, jedoch derart verstellbar verbunden, dass der senkrechte Abstand des Schallkopfes 11 von der Ebene der Öffnung 23 in Abhängigkeit von der Grösse des zu untersuchenden Busens verstellt werden kann. Dieses kann beispielsweise durch einen ebenfalls auf der Welle 6 angeordneten Elektromotor 8 geschehen.

Die Verstellung des Haltearmbereichs 10a innerhalb des Haltearms 10 erfolgt vorzugsweise hydraulisch wobei die Steuerung elektronisch erfolgt. Bei Inbetriebnahme wird der Schallkopf 11 senkrecht unter seinen Kantenbereich der Öffnung 23 gefahren. Hierbei wird in der Ausgangsstellung der Haltearm 10 derart mittels der Welle 6 gedreht, dass der Schallkopf bis nahezu in Anlage an die Abdeckung 22 kommt. Das erste Sonogrammschnittbild wird aufgenommen, während Welle 6, Haltearm 10, Haltearmbereich 10a mit Schallkopf 11 eine Schwingbewegung um etwa 180° durchführt, bis der Schallkopf 11 wiederum nahezu an die Abdeckung 22 anliegt. In der folgenden kurzen Haltepause erfolgt der vorgegebene Vorschub (oder Rückschub) des Haltearmteils 10a aus dem Haltearm 10, worauf der «Rückschwung» des Schallkopfes 11 um wiederum nahezu etwa 180°C in seine um den Vorschub veränderte Ausgangslage unter Aufnahme und Aufzeichnung eines zweiten, dem ersten parallelen Sonogrammschnittbildes. Die Steuerung des zeitlichen Ablaufs von Ausführung der Schwingbewegung und des Aus- bzw. Einfahrens des Haltearmteils 10a in/aus dem Haltearm 10 erfolgt in an sich bekannter Weise elektronisch durch Mikroprozessor. Ebenso erfolgt Aufnahme und Aufzeichnung sowie Speicherung der Sonogrammschnittbilder im übrigen in an sich bekannter Weise. Ebenfalls erfolgt bei der Aufnahme des Sonogrammbilds synchron in an sich bekannter Weise Referenzaufzeichnung der jeweiligen Position des Schallkopfes 11, wodurch die korrekte Zuordnung der jeweiligen Position des Schallkopfes 11 zum Zielorgan gewährleistet ist.

Besonders vorteilhaft ist es, gegenüber der Öffnung 23, also auf dem Grund des Behälters 21 eine Videokamera 19 zur Aufzeichnung der jeweiligen Position des Schallkopfes 11 in Bezug auf das Zielorgan angeordnet ist. Auch hierdurch kann eine zweifelsfreie Zuordnung der einzelnen Sonogrammschnittbilder in Bezug auf das Zielorgan erfolgen.

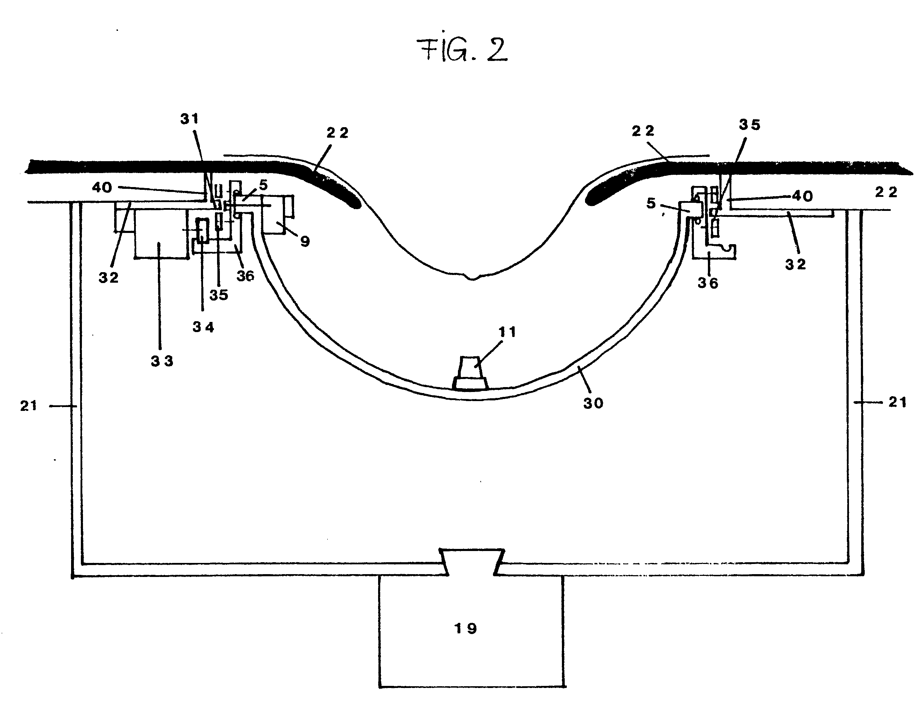

Bei der Ausführungsform gemäss Fig. 2 ist der Haltearm 30 halbkreisförmig ausgebildet und trägt in seiner Mitte den höhenverstellbaren Schallkopf 11, der senkrecht unter dem Mittelpunkt der Öffnung 23 angeordnet ist. Die beiden freien Enden des Haltearms 30 sind in Lagern 5 mittels des Motors 9 drehbar, wie zuvor beschrieben, gelagert. Der wesentliche Unterschied gegenüber der vorhergehend beschriebenen Ausführungsform besteht darin, dass die Lager ihrerseits selbst in einem sich konzentrisch um die Öffnung 23 erstreckenden Lager 40 drehbar angeordnet sind.

Dieses sich kreisförmig und konzentrisch um die Öffnung 23 erstreckende Lager 40 kann gemäss Fig. 2 ein an der Unterseite der Abdeckung 22 konzentrisch um die Öffnung 23 angebrachtes ringförmiges Bauteil mit umgekehrtem T-Profil sein, wobei auf dem in den Kreisinnenraum ragenden freien Rand des Bauteils 31 die Lager 5 beispielsweise mittels Rollen 35 drehbar und gleitend gelagert sind.

An dem nach aussen weisenden Bereich 32 des Lagerbauteils 40 ist ein Motor 33 mit einer Übertragungsvorrichtung 34, beispielsweise ein Zahnrad angebracht, mit der der halbkreisförmige Haltearm 30 mit Schallkopf 11 und den Lagern 5 angetrieben wird. Hierzu sind die Lager 5 in einem zumindest halbkreisförmig ausgebildeten Bauteil 36 mit L-Profil im Querschnitt angeordnet, das eine radial nach aussen weisende Unterkante aufweist, die zum Eingriff mit der Antriebsvorrichtung 34, beispielsweise Zahnrad, ausgebildet ist.

Durch diese Konstruktion wird erreicht, dass nicht nur der Schallkopf 11 die früher beschriebene halbkreisförmige Bewegung zur Erzeugung eines Sonogrammschnittbildes ausführen kann, sondern ausserdem wird ermöglicht, durch Drehung des halbkreisförmigen Haltearms 30 mit dem Schallkopf 11 und den Lagern 5 in dem kreisförmigen Lagerbauteil 40 um die Achse senkrecht durch den Mittelpunkt der Ebene der Öffnung 23 Serien von Sonogrammschnittbildern aufzunehmen, zu speichern etc., die nicht, wie bei Fig. 1 beschrieben, zueinander parallel, sondern in ihren Ebenen gegeneinander um vorgegebene Winkelbeträge um die Achse senkrecht durch den Mittelpunkt der Ebene der Öffnung 23 gedreht sind.

Bei Betrieb durchfährt in analoger Weise zu der Vorrichtung gemäss Fig. 1 der Haltearm 30 mit dem höhenverstellbaren Schallkopf 11 den beschriebenen Winkel von etwa 140° - 180° worauf eine vorgegebene Winkelstellung des Haltearms 30 etc. gegenüber der Ausgangsstellung erfolgt und der Haltearm 30 erneut, diesmal in entgegengesetzter Richtung, die Schwingbewegungen um etwa 180° ausführt. Der Antrieb über die Motoren 9 und 31 kann elektronisch in an sich bekannter Weise und in analoger Weise zu der gemäss Fig. 1 beschriebenen erfolgen. Gleiches gilt für Aufnahme, Aufzeichnung und Speicherung der Sonogrammschnittbilder und der jeweiligen Aufzeichnung der relativen Stellung des Schallkopfes 11 zum Zielobjekt, gegebenenfalls auch wieder durch eine am Boden des Behälters 21 gegenüber der Öffnung 23 angebrachten Videokamera 19.

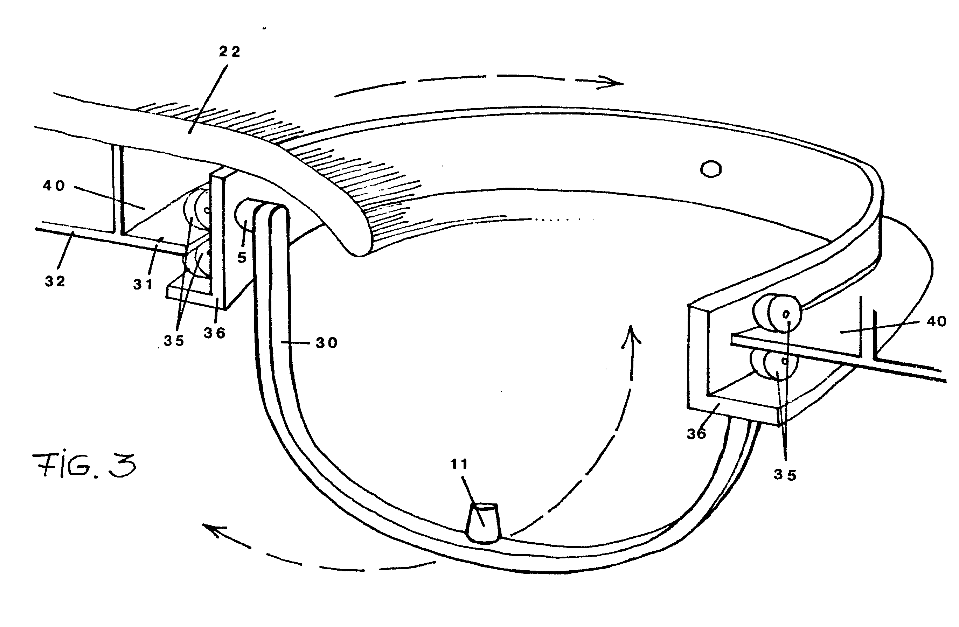

Fig. 3 gibt in einer schrägen Seitenansicht schematisch das halbkreisförmige Bauteil 36 mit L-förmigem Profil wieder, an dessen Aussenwand mehrere Paare Gleitrollen 35 angeordnet sind, über die das Bauteil 36 auf dem nach innen weisenden Rand 31 des kreisförmigen Lagers 40 drehbar ist. Der Antrieb für die Drehbewegung des Bauteils 36 ist bei dieser Darstellung nicht wiedergegeben.

In der Innenwand sind sich gegenüberliegend die Lager 5 angeordnet, in denen der Haltearm 30 mit dem Schallkopf 11 um bis zu 180° schwenkbar gelagert ist. Der Antrieb für die Schwingbewegung ist bei dieser Darstellung ebenfalls nicht wiedergegeben.

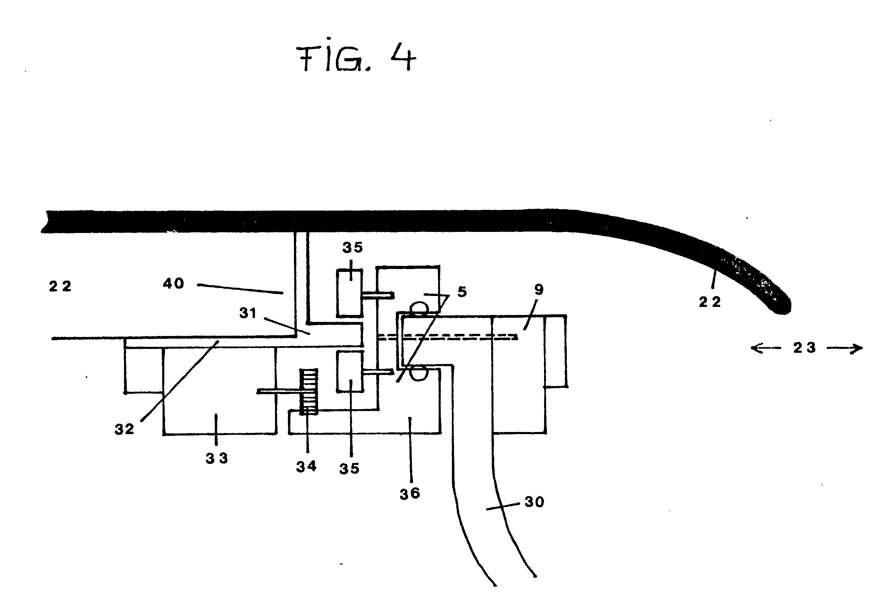

In Fig. 4 wird im Querschnitt durch den Bereich der Lager 5 und 40 der erfindungsgemässen Vorrichtung gemäss Fig. 2 schematisch die Konstruktion für die Schwingbewegung des Haltearms 30 und die Drehbewegung des kreisförmigen Bauteils 36 mit L-Profil im Querschnitt wiedergegeben. Für den Antrieb der geschilderten Schwingbewegung des Haltearms 30 ist an dessen oberem Ende im Bereich des Lagers 5 ein Elektromotor 9 angeordnet; der Antrieb des halbkreisförmigen Bauteils 36, in dem die Lager 5 des Haltearms 30 angeordnet sind, wird in dem ringförmigen Lager 40 auf dem in den Kreis hineinragenden Rand 31 durch den Elektromotor 33 über eine Antriebsvorrichtung 34, z.B. ein Zahnrad, gedreht, wobei die radial nach aussen weisende Unterkante des Bauteils 36 im L-Profil im Querschnitt zum Eingriff mit Antriebsrichtung 34 ausgebildet ist.

Bei der Ausführungsform gemäss Fig. 5 handelt es sich um eine Variation der Ausführungsform gemäss Fig. 2. Hierbei wird die Schwingbewegung des Haltearms 30 in den Lagern 5 durch den Antriebsmotor 9 in angegebener Weise konstruktionsmässig durchgeführt. Die Lager 5 sind bei dieser Ausführungsform in einem gabelförmigen Bauteil 37 angeordnet, wobei der stielartige Schaft 37a des Bauteils 37 drehbar und wasserdicht im Boden des Behälters 21, senkrecht unter dem Mittelpunkt der Ebene der Öffnung 23 gelagert ist. Der durch den Boden des Behälters 21 durchtretende Teil des Bereichs 37a steht mit einer Antriebsvorrichtung 34, beispielsweise einem durch den Motor 33 angetriebenen Zahnrad in Eingriff, so dass durch den Motor 33 die Führung des Systems um die vertikale Achse erfolgt. Das freie Ende des Bauteils 37a ist in dem Stützteil 38 gelagert. Im übrigen kommt den Bezugszeichen die vorgegebene Bedeutung zu.

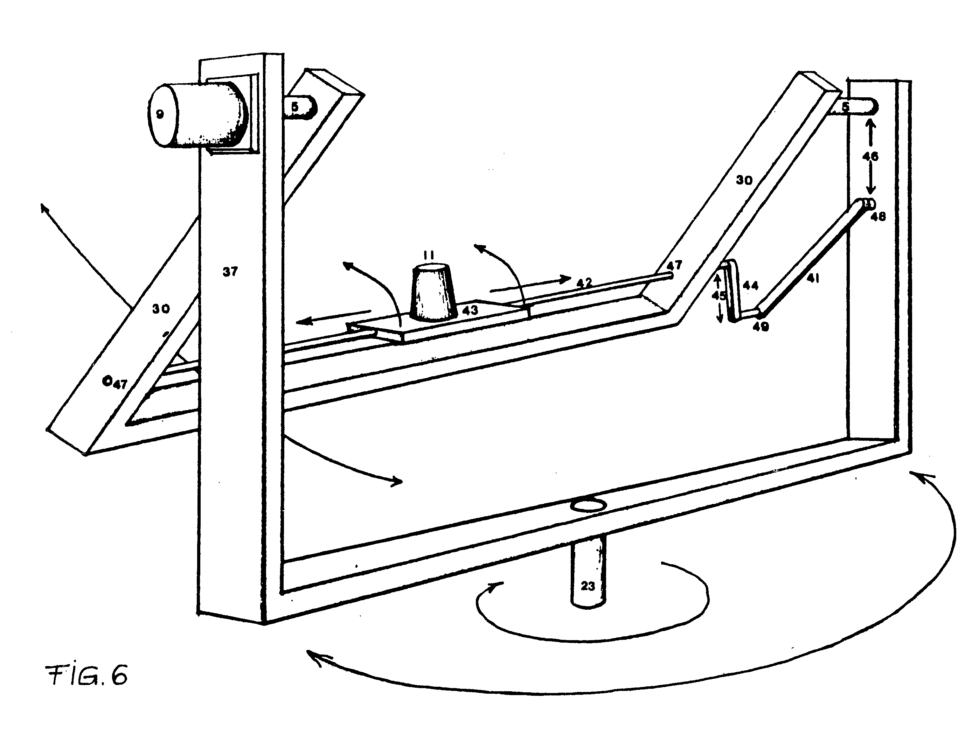

In Fig. 6 wird eine bevorzugte Ausführungsform der erfindungsgemässen Vorrichtung wiedergegeben. Hierbei handelt es sich um eine wesentliche Weiterführung der Ausführungsform gemäss Fig. 5, wobei der Schallkopf 11 nicht mehr unmittelbar auf dem Haltearm 30 angeordnet ist; vielmehr ist der Schallkopf 11 auf einer horizontal nach beiden Seiten bewegbaren Platte 43 befestigt, die wiederum auf einer Stange 42 montiert ist, die waagrecht und drehbar in den aufsteigenden Bereichen des Haltearms 30 in den Lagern 47 gelagert ist. Die Stange 42 ragt im Bereich des Lagers 47 durch den Haltearm 30 hindurch und ist über ein kurbelförmiges oder parallelogrammartiges Gestänge 44, 41 mit dem nächstliegenden aufsteigenden Arm des gabelförmigen Bauteils 37 im Lager 48 beweglich verbunden. Stange 41 ist in den Punkten 48 und 49 in Lagern beweglich angebracht.

Auf diese Weise wird erreicht, dass im Ergebnis der Schallkopf 11 um eine andere Drehachse als der Haltearm 30 gedreht wird. Die Gelenkgestängeverbindung zwischen der Stange 42 und dem aufsteigenden Bereich des gabelförmigen Bauteils 37 kann beispielsweise aus einem in Ruhestellung senkrecht ausgerichteten Gestängeschenkel 44 bestehen, der mit der Stange 42 starr verbunden ist, und mit dieser einen Winkel von beispielsweise 90° bildet. Im anderen Ende des Schenkels 44 ist eine Stange drehbar gelagert, die parallel zur Stange 42 verläuft. Das freie Ende dieser Stange ist beispielsweise über ein Kugelgelenk 49 mit dem anderen Schenkel 41 des Gelenkgestänges verbunden, dessen freies Ende, beispielsweise über ein Kugelgelenk 48, mit dem nächstliegenden aufsteigenden Bereich des gabelförmigen Bauteils 37 verbunden ist. Der senkrechte Abstand 46 dieses Verbindungspunktes zu dem Lager 5 sowie die Länge des Schenkels 44 (bei entsprechender Längenanpassung des Schenkels 41) bestimmt das Ausmass, um das sich der Drehwinkel des Schallkopfs 11 von dem des Haltearms 30 unterscheidet.

Bei Verwendung dieser Vorrichtung liegt der Kreuzungspunkt aller Schallwellen aus dem Schallkopf 11 nicht mehr zwangsläufig auf der Drehachse des Haltearms 30, wie es beispielsweise bei der Ausführungsform gemäss Fig. 5 der Fall ist, sondern kann durch entsprechende Änderungen des Abstandes 46 und der Schenkellänge 44 hinsichtlich seiner Lage variiert werden.

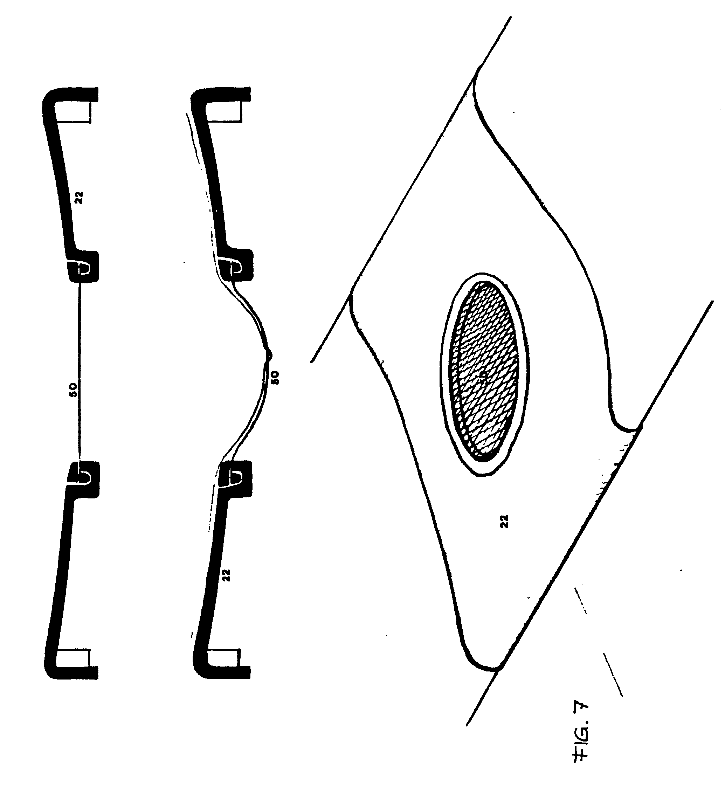

In Figur 7 wird die Abdeckung 22 wiedergegeben, in der die Öffnung mit einer Folie oder vorzugsweise mit einem Netz 50 versehen ist. Wird eine Folie verwendet, wird zur Ankopplung der Haut des Busens an die Folie entweder ein Öl oder ein handelsübliches Kontaktgel zwischen Folie und Haut aufgetragen. Ist in der Öffnung ein dünnes elastisches leicht auswechselbares Netz aus sehr feinen, den Ultraschallimpuls nicht störenden Kunststoffäden gespannt, ergibt sich hierdurch, dass der Busen nicht frei hängend im darunterliegenden Wasserbad beweglich sondern leicht komprimiert in einer unbeweglichen Position gehalten ist. Hierdurch werden Bewegungsartefakte während der Echomammographie vermieden, wobei gleichzeitig die vom Ultraschall zu durchdringende Gewebsdicke reduziert wird. Hierdurch wird der Einsatz von hochfrequenten Schallköpfen ermöglicht, die wiederum eine bessere Diagnostik von Krebs in der Brust erlauben.

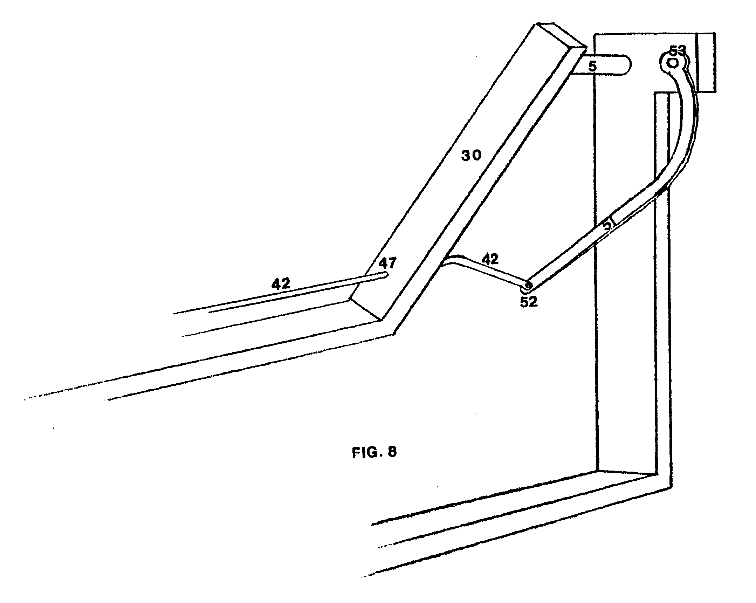

In Fig. 8 wird eine weitere Ausführungsform der Vorrichtung gemäss Fig. 6 wiedergegeben. Hierbei ist das Lager 53 (entsprechend Lager 48 in Fig. 6) im aufsteigenden Arm des gabelförmigen Bauteils 37 neben dem Lager 5 angeordnet; das Gestänge 51 (entsprechend Gestänge 41 in Fig. 6) ist gebogen ausgebildet und über ein Kugelgelenk 52 mit der

9 **0 032 751** 10

Verlängerung der Stange 42 verbunden, wobei die Verlängerung der Stange 42 durch das Lager 47 hindurch in einem Winkel von etwa 90° abgebogen ist.

### Patentansprüche

1. Mechanisches Zusatzgerät zu Compoundgeräten für Echomammographie zur Ultraschalluntersuchung der weiblichen Brust, bestehend aus einem Wasserbehälter (21) mit einer Abdeckung (22) die eine Öffnung (23) aufweist; ferner einem in den Wasserbehälter (21) unter die Öffnung (23) ragenden Haltearm (10, 30), der mindestens einen Schallkopf (11), dessen Schallfeld-Ausbreitungsvektor auf die Öffnung (23) gerichtet ist, aufweist, und der Rotationsbewegungen um wenigstens eine Achse (6) ausführen kann, dadurch gekennzeichnet, dass diese Achse (6) in der Ebene der Abdeckung (22) liegt und durch den Mittelpunkt der Öffnung (23) geht.

2. Vorrichtung nach Anspruch 1, dadurch gekennzeichnet, dass der den Schallkopf tragende Bereich des Haltearms (10a) senkrecht zur Rotationsebene des unter die Öffnung (23) ragenden Haltearms (10) verschiebbar ausgebildet ist.

3. Vorrichtung nach Anspruch 2, dadurch gekennzeichnet, dass der den Schallkopf tragende Bereich des Haltearms (10a) hydraulisch teleskopartig aus dem Haltearm (10) senkrecht zu dessen Rotationsebene ausfahrbar ausgebildet ist.

4. Vorrichtung nach mindestens einem der vorhergehenden Ansprüche, dadurch gekennzeichnet, dass der Haltearm (10) senkrecht zur Ebene der Öffnung (23) höhenverstellbar ausgebildet ist.

5. Vorrichtung nach mindestens einem der vorhergehenden Ansprüche, dadurch gekennzeichnet, dass auf dem Haltearm (10) zwei oder mehrere Schallköpfe angeordnet sind.

6. Vorrichtung nach Anspruch 5, dadurch gekennzeichnet, dass auf dem Haltearm (10) mehrere Schallköpfe unterschiedlicher Frequenzen in paralleler Schallrichtung angeordnet sind.

7. Vorrichtung nach Anspruch 6, dadurch gekennzeichnet, dass die Schallköpfe gleicher oder unterschiedlicher Frequenzen zueinander in Schallrichtung gewinkelt angeordnet sind.

8. Vorrichtung nach mindestens einem der vorhergehenden Ansprüche, dadurch gekennzeichnet, dass senkrecht unterhalb der Ebene der Öffnung (23) eine Videokamera (19) zur Aufzeichnung der jeweiligen Position der Schallköpfe während der Messungen angeordnet ist.

9. Vorrichtung nach mindestens einem der vorhergehenden Ansprüche, dadurch gekennzeichnet, dass das Lager (5) des Haltearms (10) konzentrisch um die Öffnung (23) drehbar ausgebildet ist.

10. Vorrichtung nach Anspruch 9, dadurch gekennzeichnet, dass das Lager in einer Ringschiene (36), die an der Unterseite der Abdeckung (22) konzentrisch um die Öffnung (23) angeordnet ist, drehbar ausgebildet ist.

11. Vorrichtung nach Anspruch 9, dadurch gekennzeichnet, dass das oder die Lager (5) des Haltearms (10) in einem gabelförmigen Bauteil (37) angeordnet sind, wobei der stielartige Schaft (37a) der Gabel um seine eigene Achse drehbar ist, die Drehachse senkrecht auf der Ebene der Öffnung (23) steht und durch deren Mittelpunkt geht und wasserdicht durch den Boden des Behälters (21) geführt ist.

12. Vorrichtung nach mindestens einem der vorhergehenden Ansprüche, dadurch gekennzeichnet, dass die Öffnung (23) mit abgerundeten Kanten versehen ist.

13. Vorrichtung nach mindestens einem der vorhergehenden Ansprüche, dadurch gekennzeichnet, dass in der Öffnung (23) membranartig eine von Ultraschall leicht zu durchdringende dünne, elastische Folie (50) oder ein von Ultraschall leicht zu durchdringendes Netz angeordnet ist.

14. Vorrichtung nach mindestens einem der vorhergehenden Ansprüche, dadurch gekennzeichnet, dass die Abdeckung (22) mit der Öffnung (23) als eine über dem Wasserbehälter (21) angeordnete Stahlliege für die Patientin ausgebildet ist.

15. Vorrichtung nach Anspruch 11, dadurch gekennzeichnet, dass der Schallkopf (11) auf einer horizontal verschiebbaren Platte (43) befestigt ist, die wiederum auf eine drehbare Stange (42) montiert ist, die waagerecht und drehbar in den aufsteigenden Schenkelbereichen des halbkreisförmigen oder U-förmigen Haltearms (30) gelagert ist, wobei ein Ende der drehbaren Stange (42), das in seiner Lagerung durch den Haltearm (30) hindurchtritt, über ein parallelogrammartiges Gestänge, dessen einzelne Gestängeabschnitte durch Drehgelenke miteinander verbunden sind, mit dem nächstliegenden aufsteigenden Schenkel des gabelförmigen Bauteils (37) verbunden ist.

### Claims

1. Mechanical accessory appliance for use with compound appliances for echo-mammography for the ultrasonic examination of the female breast, consisting of a water container (21) having a covering (22) which comprises an aperture (23) and further comprising a support arm (10, 30) projecting into the water container (21) below the aperture (23) and having at least one sound head (11), of which the sound field propagation vector is directed at the aperture (23), and which is capable of rotary movement about at least an axis (6), characterised in that this axis (6) lies in the plane of the covering (22) and passes through the central point of the aperture (23).

2. Device according to claim 1, characterised in that the region of the support arm (10a) which carries the sound head is constructed to be displaceable at a right-angle to the plane of rotation of the support arm (10) which projects under the aperture (23).

3. Device according to claim 2, characterised in that the region of the support arm (10a) which carries the sound head is constructed to be hydraulically extensible in telescopic fashion out of the support arm (10) and at a right-angle to the plane of rotation.

4. Device according to at least one of the preceding claims, characterised in that the support arm (10) is constructed to be adjustable in height at a right-angle to the plane of the aperture (23).

5. Device according to at least one of the preceding claims, characterised in that two or more sound heads are disposed on the support arm (10).

6. Device according to claim 5, characterised in that a plurality of sound heads of different frequencies are disposed in a parallel sound direction on the support arm (10).

7. Device according to claim 6, characterised in that the sound heads of like or different frequencies are disposed in angular relationship to one another in the sound direction.

8. Device according to at least one of the preceding claims, characterised in that perpendicularly below the plane of the aperture (23) there is a video camera (19) for recording the relevant position of the sound heads at any given time during tests.

9. Device according to at least one of the preceding claims, characterised in that the mounting (5) of the support arm (10) is constructed to be rotatable concentrically about the aperture (23).

10. Device according to claim 9, characterised in that the mounting is constructed to be rotatable in an annular track (36) disposed on the underside of the covering (22) and concentrically about the aperture (23).

11. Device according to claim 9, characterised in that the mounting or mountings (5) of the support arm (10) is/are disposed in a bifurcated component (37), the handle-like stem (37a) of the fork being rotatable about its own axis, the axis of rotation being at a right-angle to the plane of the aperture (23) and passing through the central point thereof, and being guided in watertight fashion through the bottom of the container (21).

12. Device according to at least one of the preceding claims, characterised in that the aperture (23) is provided with rounded adges.

13. Device according to at least one of the preceding claims, characterised in that disposed in the manner of a diaphragm in the aperture (23) is a thin elastic film (50) or a mesh, either of which is easily penetrated by ultrasonic waves.

14. Device according to at least one of the preceding claims, characterised in that the covering (22) with the aperture (23) is constructed as a steel lounge bed to accommodate the patient.

15. Device according to claim 11, characterised in that the sound head (11) is mounted on a horizontally displaceable plate (43) which is in turn mounted on a rotatable rod (42) which is mounted horizontally and rotatably in the rising arm portions of the semi-circular or U-shaped support arm (30), one end of the rotatable rod (42) which in its mounting passes through the support arm (30) being connected to the closest located rising arms of the bifurcated component (37) via a linkage resembling a parallelogram and of which individual linkage portions are connected to one another by swivel joints.

**Revendications**

1. Accessoire mécanique pour appareils compound d'écho-mammographie pour un examen par ultrasons des seins d'une femme, se composant d'un récipient d'eau (21) muni d'un couvercle (22) qui comporte une ouverture (23); et, un bras de retenue (10, 30) pénétrant dans le récipient d'eau (21) en dessous de l'ouverture (23), qui comporte au moins une tête ultrasonique (11), dont le vecteur d'élargissement de champ ultrasonique est dirigé vers l'ouverture (23) et qui peut exécuter des mouvements de rotation autour d'au moins un axe (6), caractérisé en ce que cet axe (6) est situé dans le plan du couvercle (22) et passe par le centre de l'ouverture (23).

2. Dispositif selon la revendication 1, caractérisé en ce que la zone du bras de retenue (10a) qui porte la tête ultrasonique est agencée de façon à pouvoir se déplacer perpendiculairement au plan de rotation du bras de retenue (10) s'engageant en dessous de l'ouverture (23).

3. Dispositif selon la revendication 2, caractérisé en ce que la zone du bras de retenue (10a) qui porte la tête ultrasonique est agencée de façon à pouvoir sortir hydrauliquement et télescopiquement du bras de retenue (10) perpendiculairement à son plan de rotation.

4. Dispositif selon au moins une des revendications précédentes, caractérisé en ce que le bras de retenue (10) est agencé de façon à être déplaçable en hauteur perpendiculairement au plan de l'ouverture (23).

5. Dispositif selon au moins une des revendications précédentes, caractérisé en ce qu'il est prévu sur le bras de retenue (10) deux ou plusieurs têtes ultrasoniques.

6. Dispositif selon la revendication 5, caractérisé en ce qu'il est prévu sur le bras de retenue (10) plusieurs têtes ultrasoniques de fréquences différentes présentant des directions de propagation parallèles.

7. Dispositif selon la revendication 6, caractérisé en ce que les têtes ultrasoniques de fréquences identiques ou différentes sont disposées avec une inclination relative par rapport à la direction de propagation.

8. Dispositif selon au moins une des revendications précédentes, caractérisé en ce qu'il est prévu, perpendiculairement en dessous du plan de l'ouverture (23), une caméra vidéo (19) pour enregistrer la position correspondante des têtes ultrasoniques pendant les mesures.

9. Dispositif selon au moins une des revendications précédentes, caractérisé en ce que le palier (5) du bras de retenue (10) est agencé de façon à pouvoir tourner concentriquement autour de l'ouverture (23).

10. Dispositif selon la revendication 9, caractérisé en ce que le palier est agencé de façon à pouvoir tourner dans un rail annulaire (36) qui est disposé sur le côté inférieur du couvercle (22) concentriquement autour de l'ouverture (23).

11. Dispositif selon la revendication 9, caractérisé en ce que le ou les paliers (5) du bras de retenue (10) sont disposés dans une pièce de forme fourchue (37), la tige en forme de manche (37a) de la fourche peut tourner autour de son propre axe, qui est orienté perpendiculairement au plan de l'ouverture (23) et qui passe par le centre de celle-ci, et elle est guidée de façon étanche à l'eau au travers du fond du récipient (21).

12. Dispositif selon au moins une des revendications précédentes, caractérisé en ce que l'ouverture (23) est pourvue de bords arrondis.

13. Dispositif selon au moins une des revendications précédentes, caractérisé en ce qu'il est prévu dans l'ouverture (23), à la façon d'une membrane, une feuille élastique mince (50) facile à traverser par des ultrasons, ou bien un filet facile à traverser par des ultrasons.

14. Dispositif selon au moins une des revendications précédentes, caractérisé en ce que le couvercle (22) muni de l'ouverture (23) est agencé, pour la patiente, sous la forme d'un support en acier à disposer au-dessus du récipient d'eau (21).

15. Dispositif selon la revendication 11, caractérisé en ce que la tête ultrasonique (11) est fixée sur une plaque pouvant coulisser horizontalement, qui est elle-même placée sur une barre tournante (42) qui est montée horizontalement et à rotation dans les parties ascendantes de branches du bras de retenue (30) en forme de demi-cercle ou en forme de U, une extrémité de la barre tournante (42), qui traverse dans sa zone de palier le bras de retenue (30), étant reliée, par l'intermédiaire d'une tringlerie en parallélogramme dont les différentes tringles sont reliées entre elles par des articulations, à la branche ascendante adjacente de la pièce de forme fourchue (37).

FIG. 1

FIG. 2

0 032 751

FIG. 3

0 032 751

FIG. 4

FIG.5

FIG.6

FIG. 7

FIG. 8

0 032 751